Europäisches Patentamt

European Patent Office

Office européen des brevets

(11) Publication number: **0 270 168**
**A2**

# (12) EUROPEAN PATENT APPLICATION

(21) Application number: 87202281.9

(51) Int. Cl.⁴: **A61B 17/56**

(22) Date of filing: 20.11.87

(30) Priority: 25.11.86 IT 2245186

(43) Date of publication of application:
08.06.88 Bulletin 88/23

(84) Designated Contracting States:
AT BE CH DE ES FR GB GR LI LU NL SE

(71) Applicant: **Ambrosio, Luigi**
**Via S. Luisa di Marillac 16**
**I-80122 Naples(IT)**

Applicant: **Nicolais, Luigi**
**Via Orazio 138**
**I-80122 Naples(IT)**

Applicant: **Huang, Samuel J.**
**10 Sumner Drive**
**Storrs Connecticut(US)**

Applicant: **Leone, Antonio**
**Via Fantoli 16/15**
**I-20138 Milan(IT)**

(72) Inventor: **Ambrosio, Luigi**
**Via S. Luisa di Marillac 16**
**I-80122 Naples(IT)**
Inventor: **Nicolais, Luigi**
**Via Orazio 138**
**I-80122 Naples(IT)**
Inventor: **Huang, Samuel J.**
**10 Sumner Drive**
**Storrs Connecticut(US)**
Inventor: **Leone, Antonio**
**Via Fantoli 16/15**
**I-20138 Milan(IT)**

(74) Representative: **Appoloni, Romano et al**
**ING. BARZANO' & ZANARDO MILANO S.p.A.**
**Via Borgonuovo 10**
**I-20121 Milano(IT)**

(54) Orthopedic prosthesis for the reduction of bone fractures, material for manufacturing it and processes to obtain them.

(57) The invention relates to an orthopedic prosthesis for the reduction of bone fractures, such as plates, pins, screws, and the like, characterized in that it is constituted by laminated composite material, on the basis of a prepreg comprising at least one resin, and a reinforcement having the form of long and continuous fibres, or by a fabric thereof, said fibres being orientated along a predetermined direction, the elastic modulus of said composite material being lower than, or equal to that of the bone, and its mechanical strength being greater than or equal to that of the bone.

## "ORTHOPEDIC PROSTHESIS FOR THE REDUCTION OF BONE FRACTURES, MATERIAL FOR MANUFAC-TURING IT AND PROCESSES TO OBTAIN THEM"

It is known that such prostheses as plates, pins and screws, presently used in orthopedics for the reduction of bone fractures are prevailingly manufactured from such metal material, as stainless steel or cobalt-chromium alloys. Such metal materials have a much higher elastic modulus than of the bones, about 10 times as that.

The greater rigidity of the metal portion causes the transmission to it of the most of the stresses the prosthesis-bone system has to face, with the consequence that the bone eventually results stress-shielded. Thus, this considerable difference in rigidity between the metal prosthesis and the bone prevents a quick healing of the bone, because of the proliferation of primary callus during the process of rejoining of the two parts of the fractured bone. At the end, the high rigidity of the metal prosthesis can cause atrophy and osteoporosis of the bone it is applied to.

A further problem is constituted by the fact that the metalprostheses can undergo corrosion phenomena.

To the purpose of solving these problems, the recent research has been addressed towards alternative materials.

Purpose of the present invention is to propose a material suitable for the manufacturing of orthopedic prostheses for the recomposition of bone fractures, which shows low enough rigidity, in view of the above discussed system, but, at the same time, high enough properties of mechanical resistance.

In order to achieve such a purpose, the present invention proposes an orthopedic prosthesis for the reduction of bone fractures, such as plates, pins, screws, and the like, characterized in that it is constituted by laminate composite material, on the basis of a prepreg comprising at least one resin and a reinforcement in the form of long and continuous fibres, or fabric thereof, said fibres being orientated along a predetermined direction, the elastic modulus of said composite material being lower than or equal to that of the bone, and its mechanical strength being greater than or equal to that of the bone.

The prosthesis of the invention, the material it is composed by, and their preparation processes can be accomplished according to different forms of practical embodiment, on condition that the above-defined characteristics are complied with.

To the purpose of illustrating some of the possible forms of practical embodiment of the invention, hereunder some practical examples thereof are described, which in no way are to be understood as being limitative thereof.

### Example 1

A plate of laminate composite material according to the invention has been prepared by using a prepreg of long and continuous carbon fibres, and high-performance epoxy resin.

The structure of the laminate is constituted by about 18 laminae of prepreg carbon fibres, a thickness of 4 mm being obtained, with structure having the following orientation, to have a symmetric and balanced laminate:

$$0$$
$$+45$$
$$-45$$
$$\underline{0}$$
$$0$$
$$-45$$
$$+45$$
$$\underline{0}$$

The so-prepared laminate is placed on a metal tool having the shape of the desired object, is enclosed in a vacuum bag for bag-moulding and, once the vacuum is applied, it is placed in an autoclave. The temperature is gradually increased up to reach 177°C, and a pressure of a few atmospheres is contemporaneously applied. The temperature is then held constant at 177°C for four hours. After cooling down, and pressure released, the bag is opened and the piece is removed. The composite plates can be also prepared by using the silicone mould, which is described in the following Examples.

Then, by using a water-jet technique, or a high-speed drill, the holes are drilled at the proper place.

The torsional rigidity of the so-produced plate is of $3.4 \times 10^{6}$ kg.mm$^2$, while the value of the same torsional rigidity is $5.36 \times 10^{6}$ kg.mm$^2$ for the bone. The resistance in flex is the same as of the bone, i.e., $1 \times 10^{4}$ kg.mm, the dimensions of the bone are D = 21 mm and d = 14 mm.


## Example 2

To produce an inframedullary pin according to the invention, a silicone mould has been used. The mould is prepared by using a metal box inside which the model pin is placed, at this point a silicone solution (E RTV) is poured, together with the proper amounts of catalyst, which will crosslink within 12 hours at room temperature. The model is subsequently removed, and in the cavity the laminate composite material on the basis of prepreg is placed. During the prepreg curing step, the silicone mould, which is contained in the metal box, tends to expand, developing such a pressure, as to prevent voids from forming inside the laminate. In this Example, a prepreg commercial carbon fabric hase been used.

The laminate is constituted by 10 laminae of orientated (0-90°) prepreg fabric:

<div align="center">

0

__90__

0

__90__

</div>

The system is then closed and transferred into an oven, wherein it is kept 3 hours at 100°C. After cooling down, the composite pin is removed from the mould, and kept in the oven for 2 hours at 180°C, to complete the cure cycle.

To the purpose of rendering the system highly biocompatible, the pin is coated with a film of polydimethylsiloxane, by casting, from a 50%-by weight solution of polydimethylsiloxane and tetrahydrofuran (THF).

The flexural properties of the composite pin are the following, M being the flexural strength, and EI being the flexural rigidity:

|  | M (kg mm) | EI (kg/mm$^2$) |
|---|---|---|
| BONE | 2928 | $2.93 \times 10^{6}$ |
| PIN | 4175 | $1.27 \times 10^{6}$ |


## Example 3

Composite screws are prepared by using high-$T_g$ resin (e.g., an epoxy resin) and continuous carbon fibres or fiberglass, and obtained by the lamination of prepregs in direction 0°, ±90° and ±70° relatively to the longitudinal section of the screw:

0

+70

-70

-- 90 --

90

-70

+70

--- 0 ---

The end shape is obtained by using the silicone mould, wherein the shape is preformed by using the method as disclosed in Example 2.

Example 4

A composite screw has been prepared according to the following manufacturing method, by bearing in mind that according to the invention, the main difficulty faced in preparing composite screws is obtaining the threads reinforced with continuous fibres, and reducing the voids content. The problem is solved with the silicone which expands during the processing cycle, developing a high enough pressure to prevent voids from being formed, and to force the fibres into the screw threads. The preparation of the screw is as follows: a small strip of prepreg fibre is rolled up, so to obtain a stick, the diameter of which is about half the diameter of the screw. Subsequently, another strip of fibre or of fabric is wound around said stick, in the direction perpendicular to the longitudinal section, and the last layer is accomplished by winding another strip of prepreg fibre in the direction of the threads. The so-prepared stick is inserted in the mould, and then it is placed in the oven for 3 hours at 100°C. After cooling down, the composite screw is removed from the mould, and transferred into the oven, wherein it is kept at 180°C for 2 hours, to complete the cure cycle.

In order to evaluate the mechanical characteristics of screws of composite material according to the invention, tests have been carried out: in particular, three different screws, made of the material according to the invention, marked as (b), (c) and (d) have been tested as compared with a metal screw of the prior art, marked as (a). The results are reported in the following Table.

## TABLE

Ultimate Strength and Specific Strength in Pure Shear Tests of Metal (a), Carbon Fiber/Epoxy (b), Kevlar Fabric/Epoxy (c), Kevlar Fabric-Carbon Fibre/Epoxy (d) Screws

| Screw Type | Weight (grams) | Ultimate Strength $(kg/cm^2)$ | Specific Strength $(kg/cm^2 \, gr)$ |
|---|---|---|---|
| a) Metal screw | 4.14 | 3536 | 854 |
| b) Carbon fibre epoxy screw | 1.04 | 2438 | 2344 |
| c) Kevlar fabric/ epoxy screw | 0.88 | 2239 | 2544 |
| d) Kevlar fabric- Carbon fibre/ epoxy screw | 0.85 | 2961 | 3484 |

It can be seen that the screws of composite material according to the invention show very much improved specific properties relatively to the metal screw: in fact, the specific resistance, in the pure shear test, of composite screw (b) made from carbon fibre/prepreg epoxy is 2.7 times higher than of the metal screw, and that of screw (d), of kevlar fabric-carbon fibre/epoxy is four times higher.

In general, the prostheses according to the invention have mechanical strength substantially greater than or equal to that of bone.

Their rigidity, or elastic modulus, is, in value, lower, or at most equal to that of the bone they have to be applied to.

## Claims

1. Orthopedic prosthesis for the reduction of bone fractures, such as plates, pins, screws, and the like, characterized in that it is constituted by laminate composite material, on the basis of a prepreg comprising at least one resin and a reinforcement in the form of long and continuous fibres, or by a fabric thereof, said fibres being orientated along a predetermined direction, the elastic modulus of said composite material being lower than or equal to that of the bone, and its mechanical strength being greater than or equal to that of the bone.

2. Prosthesis according to claim 1, characterized in that said resin is a thermoplastic resin.

3. Prosthesis according to claim 1, characterized in that said resin is a thermosetting resin.

4. Prosthesis according to claim 1, characterized in that said resin is an epoxy resin.

5. Prosthesis according to claim 1, characterized in that said fibres are carbon fibres.

6. Prosthesis according to claim 5, characterized in that said reinforcement is constituted by a whole of carbon fibres and carbon fibre fabric.

7. Prosthesis according to claim 1, characterized in that said fibres are selected from the following: graphite, glass, resin.

8. Prosthesis according to claim 1, characterized in that it is coated with biocompatible material.

9. Prosthesis according to claim 8, characterized in that said biocompatible material is polydimethylsiloxane.

10. Composite material for the production of a prosthesis according to the preceding claims.

11. Process for the manufacturing of a prosthesis according to claim 1, characterized in that it comprises the steps of (a) preparation of said laminate composite material, by means of a prepreg of at least one resin and said reinforcement in the form of long and continuous fibres, or of a fabric thereof, wherein said fibres are orientated according to a predetermined direction, (b) moulding of said laminate composite material.

12. Process according to claim 11, characterized in that it comprises a further treatment step.

13. Process according to claim 11, characterized in that said moulding step is carried out in a silicone mould.

14. Process according to claim 11, characterized in that said moulding step is accomplished by vacuum moulding.

15. Process for the preparation of a prosthesis according to claim 1, in particular of a screw, characterized in that it comprises the steps of rolling up a small strip of reinforcement in the form of a prepreg fibre so to obtain a stick, the diameter of which is lower than the diameter of the finished screw, of wounding a second strip of prepreg fibre said stick, in the direction perpendicular to the longitudinal section of this latter, wounding a further strip of prepreg fibre in the direction of the threads of the screw, and of inserting in a mould for being formed the so-prepared composite material.

16. Process according to claim 15, characterized in that said mould is of silicone.

17. Process according to claim 15, characterized in that it comprises a further treatment step.

18. Process according to claim 11, characterized in that it comprises a further step of coating said prosthesis with biocompatible material.

19. Prosthesis, composite material and processes to obtain them as hereinabove disclosed, in particular in the examples.